# EUROPEAN PATENT APPLICATION

(11) **EP 0 640 690 A1**
(43) Date of publication of application: **01.03.1995**
(21) Application number: 94306123.4
(22) Date of filing: 19.08.1994
(51) Int. Cl.: C12P 21/08, C07K 16/26, G01N 33/74, C12Q 1/32, C12N 5/20

(54) **Monoclonal antibodies specific for 5-dihydrotestosterone**

(30) Priority: 25.08.1993 US 111795
(71) Applicant: ELI LILLY AND COMPANY, Indianapolis, Indiana 46285 (US)
(72) Inventor: Baisden, Diana Kathryn, Indianapolis, Indiana 46220 (US); Yao, Raymond Che-Fong, Carmel, Indiana 46033 (US)
(74) Representative: Hudson, Christopher Mark

(57) **Abstract**

This invention provides novel monoclonal antibodies which are specific for 5-dihydrotestosterone. This invention further provides novel assay systems employing these antibodies to measure 5-α-reductase activity and determine 5-dihydrotestosterone levels.

## Description

Mammalian steroid 5-α-reductase, an enzyme present in mammalian tissues including skin, male genitalia, and prostate gland, catalyzes the conversion of the steroidal hormone testosterone to the steroidal hormone 5-dihydrotestosterone (17β-hydroxy-5α-androstan-3-one). 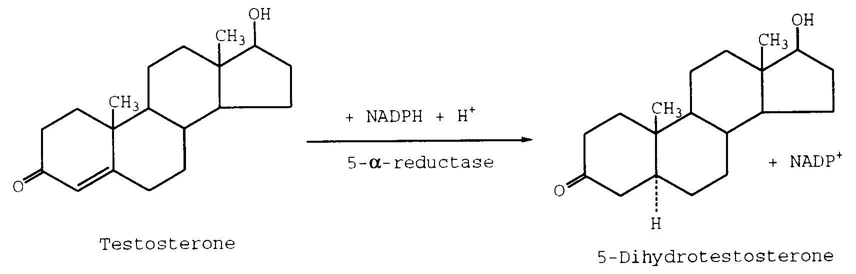
Testosterone and 5-dihydrotestosterone (5-DHT) are both androgenic hormones and they are the primary androgenic hormones in males. These steroids are primarily responsible for the physical characteristics which differentiate males from females. 5-Dihydrotestosterone, however, is much more potent than testosterone as an androgen and it acts as an end-organ effector in certain tissues, particularly in mediating growth. The formation of 5-DHT occurs primarily in the target cells themselves as a result of the reduction of testosterone by 5-α-reductase.

It is known that skin responds to androgens and is an active site of androgen metabolism. In particular, testosterone is converted to 5-DHT in the skin by the action of 5-α-reductase. Testosterone metabolism in the skin may at times be abnormally excessive and have undesirable effects as a result of the 5-DHT formed. Thus, there is considerable evidence that 5-DHT is involved in the pathogenesis of acne, including acne vulgaris, as well as other androgen-associated conditions. Price, et al., Archives in Dermatology, 111:1496 (1975). Agents which are capable of blocking the formation of 5-DHT from testosterone in skin, such as by inhibiting the activity of 5-α-reductase, would be useful in the treatment of acne.

In addition, other physical conditions and disease states, including benign prostatic hyperplasia, androgenic alopecia (common male pattern baldness), seborrhea, and female hirsutism, are also associated with elevated androgen activity and could be treated by the administration of 5-α-reductase inhibitors. See. e.g., T. Liang, et al., Endocrinology, 117:571 (1985); J.R. Brooks, et al., Steroids, 47:1 (1976); J.R. Carlin, et al., Journal of Chromatography, 427:79 (1988). Agents which are capable of blocking the formation of 5-DHT from testosterone by inhibiting the effects of 5-α-reductase would, therefore, be useful in the treatment of these conditions.

One of the many problems associated with the identification and isolation of compounds which inhibit the effects of 5-α-reductase is the absence of convenient, rapid assays which can determine the amount of the enzyme present. In the alternative, it would be greatly advantageous to have a rapid, convenient assay for determining the amount of 5-DHT in a sample as a measure of the effectiveness of an inhibitor of 5-α-reductase.

This invention provides monoclonal antibodies which are specific for 5-dihydrotestosterone, as well as immunoreactive fragments and recombinants of said monoclonal antibody. This invention also provides host cells which produce the antibodies of this invention.

The term "IC₅₀" as used herein refers to concentration of a compound which inhibits by 50% the activity being measured. The term "IC₁₀₀" as used herein refers to concentration of a compound which inhibits 100% of the activity being measured.

As used herein the term "antibody" encompasses antibodies, fragments of antibodies (such as, but not limited, to Fab, Fab', F(ab')₂,and Fv fragments), and chimeric, humanized, veneered, resurfaced, or CDR-grafted antibodies capable of binding antigens of a similar nature as the parent antibody molecule from which they are derived. The instant invention also encompasses single chain polypeptide binding molecules.

The term "antibody" as used in this document is not limited by the manner in which the antibodies are produced, whether such production is in situ or not. The term "antibody" as used in this specification encompasses those antibodies produced by recombinant DNA technology means including, but not limited, to expression in bacteria, yeast, insect cell lines, or mammalian cell lines. In one embodiment this invention encompasses host cells capable of producing the antibodies of this invention. Preferred host cells include those of murine, yeast, insect, and bacterial origin. Especially preferred are those host cells of murine origin, particularly hybridomas.

The production of antibodies, both monoclonal and polyclonal, in animals, especially mice, is well known in the art. See. e.g., C. Milstein, Handbook of Experimental Immunology, (Blackwell Scientific Pub., 1986); J. Goding, Monoclonal Antibodies: Principles and Practice, (Academic Press, 1983). For the production of monoclonal antibodies the basic process begins with injecting a mouse, or other suitable animal, with an immunogen. The mouse is subsequently sacrificed and cells taken from its spleen are fused with myeloma cells, resulting in a hybridoma that reproduces in vitro. The population of hybridomas is screened to isolate individual clones, each of which secretes a single antibody species, specific to the immunogen. The individual antibody species obtained in this way is each the product of a single B cell from the immune animal generated in response to a specific antigenic site recognized on the immunogenic substance.

After the different hybridoma cell lines are screened to identify those that produce antibody to the desired antigen, the antibodies produced by the individual hybridoma cell lines are screened to identify those having the highest affinity for the immunogenic substance, in this case 5-dihydrotestosterone, while still maintaining a low affinity for testosterone. Preferably, the monoclonal antibodies selected will have an affinity of at least about 10⁸ liters/mole, and more preferably, an affinity of at least about 10⁹ liters/mole.

Numerous species including rats, mice, rabbits, goats, and humans are useful as a source of immunized lymphocytes for the fusion protocols utilized to generate hybridomas. BALB/c mice are especially preferred as the source of the immune cells for production of the antibodies of this invention.

Treatment of the antibodies with proteolytic enzymes, such as papain and pepsin, generates antibody fragments, such as the Fab and F(ab')₂ species, which are encompassed within the scope of this invention. Treatment of the antibodies of this invention with such enzymes can therefore be used to generate the 5-dihydrotestosterone binding fragments of the invention.

Those skilled in the art will recognize that the antigen-binding region of the antibodies or antibody fragments of this invention is a key feature of the invention. The hybridoma producing the MoAb 276-1 antibodies serves as a preferred source of DNA that encodes one such antigen-binding region of the invention. This DNA, through recombinant DNA technology, can be covalently linked to DNA that encodes any desired amino acid residue sequence to yield a novel hybrid DNA sequence encoding a novel hybrid protein. Variations of this technology can be used to produce chimeric, humanized, veneered, resurfaced, and CDR-grafted antibodies capable of binding antigens of a similar nature as the parent antibody molecule from which they are derived

Chimeric antibodies are described in U.S. Patent No. 4,816,567, which issued March 28, 1989 to S. Cabilly, et al. This reference discloses methods and vectors for the preparation of chimeric antibodies. The entire contents of U.S. Patent No. 4,816,567 are herein incorporated by reference. An alternative approach to production of genetically engineered antibodies is provided in U.S. Patent No. 4,816,397, which also issued March 28, 1989 to M. Boss, et al., the entire contents of which are herein incorporated by reference. The Boss patent teaches the simultaneous co-expression of the heavy and light chains of the antibody in the same host cell.

The approach of U.S. Patent 4,816,397 has been further refined as taught in European Patent Publication No. 0 239 400, which published September 30, 1987. The teachings of this European patent publication (Winter) are the preferred format for the genetic engineering of the reactive monoclonal antibodies of this invention. The Winter technology involves the replacement of complementarity determining regions (CDRs) of a human antibody with the CDRs of a murine monoclonal antibody thereby converting the specificity of the human antibody to the specificity of the murine antibody which was the source of the CDR regions.

Single chain antibody technology is yet another variety of genetically engineered antibody which is now well known in the art. See, e.g. R.E. Bird, et al., Science 242:423-426 (1988); PCT Publication No. WO 88/01649, which was published 10 March 1988. The single chain antibody technology involves joining the binding regions of heavy and light chains with a polypeptide sequence to generate a single polypeptide having the binding specificity of the antibody from which it was derived.

The aforementioned genetic engineering approaches provide the skilled artisan with numerous means to generate molecules which retain the binding characteristics of the parental antibody while allowing the practitioner to alter the remainder of the molecule. Such techniques are especially useful in the production of bi-specific antibodies. These bi-specific antibodies have specificity for 5-dihydrotestosterone as well as for another antigen, such as a signalling moiety. Alternatively these techniques allow the production of chimeric molecules in which the antigen-recognizing region from an antibody of this invention is covalently linked to an enzyme, such as β-galactosidase, which is a signalling moiety.

The antibodies of the present invention must demonstrate a greater specificity for 5-dihydrotestosterone than for testosterone. In a preferred embodiment the antibodies of this invention demonstrate less than 25% cross-reactivity with testosterone when the specificity for 5-dihydrotestosterone is normalized to 100%. In an especially preferred embodiment the antibodies of this invention demonstrate less than 12.5% cross-reactivity with testosterone.

The usual method of quantifying specificity of an antibody is by way of a competitive ELISA assay, a technique which is well known to one of skill in the art. In such an assay the reactivity is determined by binding an avidin/5-DHT complex to a support, such as a well in a microtiter plate, as described infra. A limited amount of anti-5-DHT antibody (such as MoAb 276-1) is then added simultaneously with differing concentrations of 5-dihydrotestosterone, which competes with the bound 5-DHT for the antibody. Unbound antibody is then removed from the wells. The concentration of 5-dihydrotestosterone which inhibits 50% of the binding of the antibody is designated as the IC₅₀. The experiment is run in parallel using testosterone in place of the 5-dihydrotestosterone. The IC₅₀ of the testosterone is then compared with the IC₅₀ of the 5-dihydrotestosterone to give a relative binding affinity.

The most preferred antibody of this invention, MoAb 276-1, has an IC₅₀ of 537 nM for 5-dihydrotestosterone, while an IC₅₀ of 4568 nM was obtained for testosterone. This antibody, therefore, is 8.5 times more specific for 5-dihydrotestosterone than for testosterone. The hybridoma producing the monoclonal antibody MoAb 276-1 is available from the American Type Culture Center (ATCC) in Rockville, Maryland, 20852 under accession number ATCC HB 11410.

The present invention encompasses all antibodies reactive in a specific manner with 5-dihydrotestosterone regardless of species of origin or immunoglobulin class or subclass designation including IgG, IgA, IgM, IgE, and IgD. The most preferred antibodies of the present invention are those of the IgG class.

The antibodies of this invention can be constructed and isolated by immunization, preparation of hybridomas, and identification of antibodies with a selective reactivity to 5-dihydrotestosterone.

This invention also provides a rapid, convenient assay method for the determination of the amount of 5-dihydrotestosterone in a sample, the assay comprising an immunological assay employing an antibody specific for the dihydrotestosterone.

This invention further comprises a process for measuring the effectiveness of an inhibitor of the enzyme 5-α-reductase which comprises
a. adding a known amount of purified 5-α-reductase to a container system;
b. performing a first incubation with the 5-α-reductase and the inhibitor of the enzyme 5-α-reductase;
c. incubating a known aliquot of testosterone with the mixture of the 5-α-reductase and the inhibitor of the enzyme 5-α-reductase;
d. measuring the amount of dihydrotestosterone in the reaction mixture by employing an antibody specific for the 5-dihydrotestosterone; and
e. detecting reduction in the level of dihydrotestosterone relative to a control in which no inhibitor of the enzyme 5-α-reductase was added.

The general methodology of the assays of this invention first entails the immobilization of the target 5-dihydrotestosterone to a solid support which comprises or is contained within a container system. Such support can be particles within a container (such as a bead or a membrane). In a preferred embodiment the support is the inner surface of the container itself. The characteristics of the container system employed depend upon the type of signaling moiety used. For example, the use of a radioactive signal requires the container to be pervious to the emitted particle. When chromogenic or fluorogenic signals are used, the container system must either be transparent at the wavelength employed to detect said signal or it must be constructed in such a way as to allow a direct viewing of the reaction fluid.

The preferred system employs a plate of wells, such as those commonly used for microtiter assays or tissue culture. The most preferred system for chromogenic or fluorogenic signals employs those plates which have 96 wells and have flat bottoms to the wells. A most preferred embodiment of this invention employs microtiter plates from commercial sources which have been pre-treated so as to activate the walls of the wells to readily bind ligands.

Another method of affixing the 5-dihydrotestosterone to the solid support is by way of a linker. A frequently employed linker is a protein. A preferred protein for this purpose is avidin. One frequently employed method of binding 5-dihydrotestosterone to a protein employs the conjugation of 5-dihydrotestosterone to avidin using cross-linkers which are well known in the art. Such cross-linking reagents couple the hydroxy at the 17 position with a reactively available amino group in the protein. A preferred cross-linking protocol employs a N-hydroxysulfosuccinimide-enhanced EDC [1-ethyl-3-(3-dimethylaminopropyl)carbodiimide] procedure. J.V. Staros, et al., Analytical Biochemistry, 156:220-222.

The conjugated 5-DHT/protein complex is then immobilized on the solid support by way of hydrophobic interaction (facilitated by the use of a coating buffer such as carbonate-bicarbonate buffer at pH 9.6). Alternatively the 5-DHT/protein complex is bound to the solid support by way of a reactively available side chain using standard techniques.

The 5-dihydrotestosterone/linker conjugate can be affixed to the support surfaces either directly or indirectly. The indirect method of affixing the target employs a ligand/receptor reaction. A ligand is bound to the support surfaces. The receptor, covalently linked to the target 5-dihydrotestosterone, is then introduced into the containers. The ligand/receptor interactions then serve to affix the 5-dihydrotestosterone to the support surfaces. Preferred ligand/receptor pairs include biotin and streptavidin, biotin and avidin, and sugars and lectins.

The use of biotin and avidin or biotin and streptavidin as the ligand/receptor pair is greatly preferred because of their extremely high affinity interaction (K_{d} = 10⁻¹⁵). Avidin is a glycoprotein which naturally exists as a tetramer containing four identical subunits, each with a molecular weight of 15,000 daltons. It is readily isolatable from egg whites. See, e.g., M. Wilchek and E.A. Bayer, Analytical Biochemistry, 171:1-32 (1988) and references therein.

An especially preferred embodiment of this invention employs streptavidin in place of egg-white avidin. Streptavidin is often preferred over avidin for several reasons. Streptavidin, isolated from culture filtrates of a Streptomyces species, differs from avidin in having no carbohydrate moieties and a pI of 5.0, compared to an avidin pI of 10.0. This is important because the presence of carbohydrates and high pI values can result in increased non-specific binding. A deglycosylated form of egg-white avidin may also be used to decrease background binding. Y. Hiller, et al., Biochemical Journal, 248:167-171 (1987).

The antibodies of the current invention are capable of generating a detectable signal. The type of detectable signal employed is left to the discretion of the practitioner. The signal can be chromogenic, fluorescent, radioactive, electrical, or magnetic.

In a preferred embodiment the detectable signal is detected by spectrophotometric techniques. In an especially preferred embodiment an enzyme is linked to an antibody. The antibody to which the enzyme is linked can be either the antibody specific for the 5-dihydrotestosterone, or it can be a second antibody which is specific for the anti-5-dihydrotestosterone antibody.

As an example, the enzyme alkaline phosphatase (EC 3.1.3.1) can be easily linked to an antibody containing a reactively available thiol group. The alkaline phosphatase is generally conjugated to the antibody through a heterobifunctional cross-linker such as 4-(N-maleimidomethyl)cyclohexane-1-carboxylic acid-N-hydroxysuccinimide ester. S. Yoshitake, et al., Journal of Biochemistry, 92:1413 (1982).

For alkaline phosphatase, the signal is generated by the addition of a chromogenic substrate such as *p*-nitrophenylphosphate. Hydrolysis of *p*-nitrophenylphosphate results in release of the p-nitrophenol group, causing an increase in the absorbance at 405 nm (A₄₀₅) and appearance of a yellow color.

Other examples of preferred enzymes capable of generating a chromogenic signal are: horseradish peroxidase, β-D-glucoronidase, β-D-galactosidase, 6-phospho-β-D-galactoside 6-phosphogalactohydrolase, α-D-galactosidase, β-D-glucosidase, α-amylase, α-glucosidase, neuraminadase, butyrate esterase, lipases, acid phosphatase, pyroglutamyl aminopeptidase, L-alanine aminopeptidase, arylaminopeptidases, coagulose, urease, luciferase, glucose oxidase, and other peroxidases.

The substrate employed as well as the wavelength at which the absorbance is read is determined by the particular enzyme used to generate the signal. The type of enzyme employed may be determined in part by the constitution of the test system. For example, it would be unwise to use a peptidase as it may cleave one or more of the antibody, the linker binding the 5-dihydrotestosterone to the support, or the second antibody.

Other preferred means of generating a signal involve the use of fluorogenic substrates and radioactive signals. Fluorogenic substrates conjugated (directly or indirectly) to the antibody provide a rapid means of quantifying the antibody present. Examples of fluorogenic substrates include fluorescein isothiocyanate, Texas Red, rhodamine isothiocyanate, sulforhodamine, luciferase, eosin isothiocyanate, dansyl chloride, trifluoromethylcoumarin, phthaldialdehyde, quinolizino-substituted fluorescein isothiocyanate, and tetramethylrhodamine isothiocyanate. A fluorochrome can be incorporated into an antibody through a variety of means, taking advantage of a reactive thiol, hydroxy, or carbonyl group.

Rapid quantitation of the fluorescence can be performed using a commercially-available fluorometer (spectrofluorometer) capable of reading microtiter plates. The wavelengths of the emitting light and the receiver filter depend upon the particular fluorogenic substrate employed.

Isotopes of elements such as indium, lead, rhenium, technicium, iodine, gallium, leutecium, astatine, bismuth, boron, platinum, silver, cobalt, yterbium, ruthenium, mercury, scandium, carbon, bromine, hydrogen, phosphorous, sulfur, and yttrium may be used to radioactively label antibodies of the invention. Preferred examples of radioisotopes used as signaling moieties are phosphorous-32, iodine-125, carbon-14, tritium, indium-111, iodine-131, and sulfur-35. The method of linking the radioactive signal to the antibody depends upon the radioisotope employed and whether there is direct or indirect attachment of the signaling moiety and the antibody. For example, iodine-125 labeling of proteins such as antibodies is well known in the art. See, e.g., David, et al., Biochemistry, 13:1014-1021 (1974).

One of the steps required in the assays of the present invention involves removal of unbound antibody. The normal means of accomplishing this task is by repeatedly washing the container system with physiological saline and removing the saline by suction.

The assays of the instant invention are of particular value in determining the amount of 5-dihydrotestosterone in a sample, especially a biologicaly sample. Examples of such biological fluids include: blood or serum; cerebrospinal fluids; lymphatic fluids; saliva,; and urine. In a most preferred embodiment of this invention urine is used as the biological fluid. These assays are of particular value in monitoring the effectiveness of inhibitors of 5-α-reductase as well as the compliance of patients taking such inhibitors.

The following Examples further illustrate the present invention. Particular materials, species, and conditions employed are intended to be further illustrative of the invention and are not limiting upon the reasonable scope thereof.

### Example 1

Production of MoAb 276-1 Antibodies.

Vials of frozen hybridomas producing the MoAb 276-1 antibodies can be obtained from the American Type Culture Collection, Rockville, Maryland under the accession number HB 11410. Viable cells are recovered by thawing vial contents in a 37°C water bath while swirling the vial to faciitate rapid and uniform thawing. The cell suspension is diluted 1:2 with Balanced Salt Solution (BSS) (GIBCO-BRL) and centrifuged at 200 x g through a serum underlay to partition from the cryogenic media. Following aspiration of material above the cell pellet, cells are harvested, diluted in tissue culture media supplemented with serum and antibiotics as is common in the art and established in cell culture under standard conditions (37°C and 5% carbon dioxide). During cell culture, it is advisable to maintain the cells at concentrations of 1 x 10⁵ - 1 x 10⁷ cells/ml although modest variances are well tolerated.

MoAb 276-1 antibodies may be recovered from tissue culture in microgram per milliliter quantities. Alternatively, antibody production may be more productively pursued by establishing the hybridomas as ascites tumors in rodent species. Antibody production and harvesting methods are described in detail by Gaflre and Milstein in "Methods in Enzymology" Vol. 73B (Langone and Van Vunakis, eds. 1981) pp. 43-45.

### Example 2

Preparation of the F(ab')₂ fragments of MoAb 276-1.

The F(ab')₂ fragment of MoAb 276-1 is prepared by adding 2.4 ml of pepsin solution, containing 12.6 mg/ml of pepsin, to 1.5 g of MoAb 276-1 antibody in 270 ml of physiologically buffered saline. The mixture is held at 37°C for about 2 to three hours, and then the reacion is stopped by the addition of triethanolamine. The product is then concentrated by chromatography on a Sepharose Fast Flow® system, eluting with 0.15 M sodium acetate. The F(ab')₂-containing fractions are combined, and concentrated by dialysis to obtain 100 ml of product solution containing 992 mg of the F(ab')₂ fragment of the MoAb 276-1 antibody.

### Example 3

Assay for measuring the activity of 5-α-reductase.

In a well of a 96 well microtiter plate was admixed 20 µl of a 1.25 µg/ml solution of testosterone, 150 µl of potassium phosphate buffer (pH 6.5), 20 µl of 5-α-reductase enzyme (0.86 µg) from a female rat liver preparation, and 10 µl of β-nicotinamide adenine dinucleotide phosphate, reduced form (0.77 mg/ml solution). This reaction mixture was incubated at 37°C for 20 minutes. The enzyme was then inactivated by incubating the reaction mixture at 80°C for 10 minutes.

One hundred microliters of the above reaction mixture was then transferred to a well precoated with avidin-5-dihydrotestosterone. In additon, serial dilutions of the reaction mixture were made and 100 µl of each of these dilutions was added to separate wells. To each well was added 100 µl (0.35 µg) of MoAb 276-1. This plate was then incubated at 37°C for 2 hours.

After this incubation period, the plate was washed four times with phosphate buffered saline (PBS). After the washing, 200 µl of anti-mouse antibody/alkaline phosphatase conjugate was added to each well. This was then incubated at 37°C for 1 hour. After the incubation each well was washed four times with PBS.

To each well was then added 200 µl of p-nitrophenylphosphate. This reaction was then incubated for one hour at 37°C. Spectrophotometric measurement of each well was then performed, reading at 410 nm. This measurement provided the baseline activity of 5-α-reductase activity without an inhibitor.

### Example 4

Assay for measuring the effectiveness of an inhibitor of 5-α-reductase.

An assay similar to Example 3 was performed except that varying amounts of the compound trans-dl-4,8-dimethyl-1,2,3,4,4a,5,6,10b-octahydrobenzo-[f]quinolin-3-one 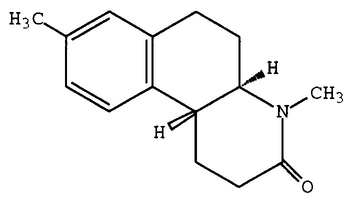
were added to the mixture of the testosterone and a known amount of the enzyme in individual wells. This compound was prepared as described in U.S. Patent 5,239,075, issued August 24, 1993, which is herein incorporated by reference.

The A₄₁₀ determined with the different concentrations of inhibitor was then compared with a reaction well in which no inhibitor was added.In one such experiment this compound was found to have an IC₁₀₀ of 0.3 µM.

### Example 5

Assay for measuring the amount of 5-dihydrotestosterone in a sample.

Each well of a 96 well, high binding, flat bottom, microtiter plate is pre-coated by the addition of 200 µL of a 15 µg/mL solution of avidin/5-dihydrotestosterone conjugate in 0.05 M carbonatebicarbonate buffer (pH 9.6). This pre-coating reaction is allowed to incubate overnight at 4°C. The plates are then washed once with distilled water to remove unbound avidin/5-DHT complex.

To each well is then added 100 µl (0.35 µg) of MoAb 276-1. This plate is then incubated at 37°C for two hours. After the incubation, unbound antibody is then removed by washing each well four times with phosphate buffered saline followed by aspiration.

After the washing, 200 µl of anti-mouse antibody/alkaline phosphatase conjugate is added to each well. This is then incubated at 37°C for 1 hour. After the incubation each well is washed four times with PBS.

To each well is then added 200 µl of p-nitrophenylphosphate. This reaction was then incubated for one hour at 37°C. Spectrophotometric measurement of each well is then performed, reading at 410 nm. The reduction in the A₄₁₀ relative to a control in which no urine sample is added indicates the amount of 5-dihydrotestosterone in the urine sample.

### Culture Deposits

Under the provisions of the Budapest Treaty on the International Recognition of the Deposit of Microorganisms for Purposes of Patent Procedures, the following culture has been deposited with the permanent culture collection of the American Type Culture Collection (ATCC) in Rockville, Maryland, 20852:

| Deposited Material | Date of Deposit | Accession Number |
|---|---|---|
| Hybridoma 276-1 | July 20, 1993 | ATCC HB 11410 |

## Claims

1. A monoclonal antibody specifically reactive with 5-dihydrotestosterone.

2. A monoclonal antibody as claimed in Claim 1 wherein the monoclonal antibody exhibits greater than four times more specificity for 5-dihydrotestosterone than for testosterone.

3. A monoclonal antibody as claimed in Claim 1 wherein the monoclonal antibody exhibits greater than eight times more specificity for 5-dihydrotestosterone than for testosterone.

4. A monoclonal antibody as claimed in Claim 3 which is MoAb 276-1, or a derivative thereof.

5. A process for measuring the amount of 5-dihydrotestosterone in a sample which comprises:
a. fixing 5-dihydrotestosterone to a solid support which comprises or is contained within a container system;
b. admixing with an aliquot of the sample a known quantity of a monoclonal antibody as claimed in any one of Claims 1 to 4, said monoclonal antibody being capable of generating a detectable signal;
c. incubating a known amount of the mixture of the sample and the monoclonal antibody in the container;
d. removing unbound antibody;
e. generating said detectable signal; and
f. measuring reduction in said detectable signal relative to a control in which no sample had been added.

6. A process for measuring the effectiveness of an inhibitor of the enzyme 5-α-reductase which comprises:
a. adding a known amount of purified 5-α-reductase and nicotinamide adenine dinucleotide phopshate to a container system;
b. performing a first incubation with the enzyme 5-α-reductase and the inhibitor;
c. incubating a known aliquot of testosterone with the mixture of the enzyme 5-α-reductase and the inhibitor;
d. measuring the amount of dihydrotestosterone in the reaction mixture by
(i) fixing to a solid support which comprises or is contained within a container system a known quantity of 5-dihydrotestosterone,
(ii) admixing with an aliquot of the mixture of the enzyme 5-α-reductase and the inhibitor, a known quantity of a monoclonal antibody as claimed in any one of Claims 1 to 4, said monoclonal antibody being capable of generating a detectable signal;
(iii) incubating a known amount of the mixture of the enzyme 5-α-reductase, the inhibitor, and the monoclonal antibody in the container;
(iv) removing unbound antibody;
(v) generating said detectable signal; and
e. measuring reduction in said detectable signal relative to a control in which no inhibitor of the enzyme 5-α-reductase was added.

7. A process as claimed in any one of Claims 5 and 6 wherein said monoclonal antibody is capable of generating a detectable signal by way of a second antibody.

8. A host cell which produces a monoclonal antibody as claimed in any one of Claims 1 to 4.

9. A hybridoma as claimed in Claim 8 which is ATCC HB 11410.
